# EUROPEAN PATENT SPECIFICATION

(11) **EP 2 751 283 B1**
(45) Date of publication and mention of the grant of the patent: **08.03.2017**
(21) Application number: 12756154.6
(22) Date of filing: 30.08.2012
(51) Int. Cl.: C12Q 1/68

(54) **PROCESS FOR SEPARATION OF OLIGONUCLEOTIDE OF INTEREST FROM A MIXTURE**
VERFAHREN ZUR TRENNUNG EINES BESTIMMTEN OLIGONUKLEOTIDS AUS EINER MISCHUNG
PROCÉDÉ DE SÉPARATION D'UN OLIGONUCLÉOTIDE CIBLE D'UN MÉLANGE

(30) Priority: 02.09.2011 GB 201115218
(43) Date of publication of application: 09.07.2014
(73) Proprietor: Glaxo Group Limited, Brentford, Middlesex TW8 9GS (GB)
(72) Inventor: DOUILLET, Nathalie, Stevenage Hertfordshire SG1 2NY (GB); FREEBAIRN, Keith, Stevenage Hertfordshire SG1 2NY (GB); GUZLEK, Hacer, Stevenage Hertfordshire SG1 2NY (GB); HUBERT, Jane, F-51097 Reims Cedex (FR); RENAULT, Jean-Hughes, F-51097 Reims Cedex (FR); THICKITT, Christopher, Stevenage Hertfordshire SG1 2NY (GB)
(74) Representative: Povey, Alexander W.G.
(86) International application number: PCT/EP2012/066842
(87) International publication number: WO 2013/030263

(56) References cited:
- WO-A2-2005/118527
- MARUYAMA TATSUO ET AL: "Sequence-selective extraction of single-stranded DNA using DNA-functionalized reverse micelles.", CHEMICAL COMMUNICATIONS (CAMBRIDGE, ENGLAND) 21 NOV 2007, no. 43, 21 November 2007 (2007-11-21), pages 4450-4452, XP002690271, ISSN: 1359-7345

## Description

This invention relates to a novel process, in particular to a process for the separation of oligonucleotides from chemical impurities using support-free liquid-liquid chromatography.

Oligonucleotides comprise relatively short sequences of nucleic acid polymers, which may be DNA or RNA, typically with fifty or fewer bases, though oligonucleotides with up to ca. 200 bases can now be synthesised. Oligonucleotides are useful in therapy. A synthetic RNA oligonucleotide, identified by the company Prosensa under their designation PRO051 and under the present applicant's designation GSK2402968 has the sequence 5'-uca agg aag aug gca uuu ca-3'. This oligonucleotide is an antisense oligonucleotide, i.e. a single strand of DNA or RNA that is complementary to a chosen sequence. It is believed to induce exon skipping of exon 51, and is under consideration in a Phase III clinical study for treatment of ambulant boys with Duchenne Muscular Dystrophy (DMD).

A problem with such oligonucleotides is their purification from impurities. For example the above-mentioned GSK2402968 is a 20-mer, and during the course of its synthesis it can become contaminated with impurities, including other oligonucleotides including shortmers and longmers, for example containing 17, 18, 19 and 21 bases. Other impurities may also be present.

It is desirable to provide a process for separation of such oligonucleotides which is efficient and applicable on an industrial production scale. Maruyama et al: "Sequence-selective extraction of single-stranded DNA using DNA-functionalised reverse micelles", Chemical Communications (Cambridge, England) No. 43, 21 Nov 2007, pages 4450-4452 discloses purification of an ologonucleotide from a mixture using displacement from one liquid phase to another by addition of a complementary hybridizing oligoncleotide. *"*Therapeutic oligonucleotides: The state of the art in purification technologies" Sanghvi et. al. Current Opinion in Drug Discovery (2004) Vol. 7 No. 8 reviews processes used for oligonucleotide purification. The literature discloses other processes. Various processes have been used for purification of DNA and RNA sequences. WO-A-01/55160 discloses purification of oligonucleotides by forming imine linkages with contaminants then removing the imine-linked impurities with chromatography or other techniques. *"*Size Fractionation of DNA Fragments Ranging from 20 to 30000 Base Pairs by Liquid/Liquid chromatography" Muller et al. Eur. J. Biochem (1982) 128 - 238 discloses use of a solid column of microcrystalline cellulose on which has been deposited a PEG/dextran phase for separation of nucleotide sequences. *"*Separation and identification of oligonucleotides by hydrophilic interaction chromatography." Easter et. al. The Analyst (2010); 135(10) discloses separation of oligonucleotides using a variant of HPLC employing a solid silica support phase. *"*Fractionation of oligonucleotides of yeast soluble ribonucleic acids by countercurrent distribution" Doctor et al. Biochemistry (1965) 4(1) 49-54 discloses use of a dry solid column packed with dry DEAE-cellulose. *"*Oligonucleotide composition of a yeast lysine transfer ribonucleic acid" Madison et al; Biochemistry, 1974, 13(3) discloses use of solid phase chromatography for separation of nucleotide sequences. The Wikipedia article at http://en.wikipedia.org/wiki/Hydrophilic interaction chromatography discloses use of hydrophilic interaction chromatography for separation of biomolecules using a solid silica substrate.

Liquid-liquid chromatography is a known separation method. Liquid-liquid chromatography uses a biphasic liquid system, comprising a stationary liquid phase maintained in an elongate column (analogous to the solid phase deployed in a tubular column in conventional chromatography), and a mobile liquid phase which is caused to flow through the column in contact with the stationary phase. During such flow, substances will partition between the mobile and stationary phases in a manner analogous to conventional solid-phase chromatography. In liquid-liquid chromatography techniques the mobile phase elutes the substances to be separated in fractions analogous to conventional liquid-solid phase chromatography. In liquid-liquid chromatography the process is normally operated support-free, i.e. no solid support matrix is required to support the liquid phases, which are maintained in their relationship in contact with each other during the process by means of the centrifugal force within the rotating column. "Countercurrent Chromatography - The Support- Free Liquid Stationary Phase" Billardello, B.; Berthod, A; Wilson & Wilson's Comprehensive Analytical Chemistry 38; Berthod, A., Ed.; Elsevier Science B.V.: Amsterdam (2002) pp 177-200 provides a useful general description of liquid-liquid chromatography. WO2005/11857 discloses use of liquid-liquid chromatography for oligonucleotide purification.

Various liquid-liquid chromatography techniques are known.

One technique is liquid-liquid counter current chromatography (termed herein "CCC"). In a CCC apparatus a circular e.g. helical or spiral tubular column of generally constant bore is rotated both around its circular centre and about a rotation axis displaced from its centre, i.e. so-called planetary rotation. This combined motion creates oscillating centrifugal force fields in the column which results in mixing and de-mixing zones along the column. A typical CCC apparatus is disclosed in WO-A-03/086639 (Brunel University).

Another known technique is centrifugal partition chromatography (termed herein "CPC"). US-A-6,537,452 and WO-A-2010/059715 suggest the possibility of use of CPC for separation of biological molecules. "High Performance Centrifugal Partition Chromatography" 2005 (online: http://web.archive.org/web/20050208201013/http://everseko.co.ip/p02.html) and "Applications of Liquid-Liquid Chromatography Instrumentation for Laboratory Preparative & Process Chemistry" Brown et al. Chromatography Today (Nov-Dec 2009) 16-19 are general articles on CPC. "Anion-Exchange Displacement Centrifugal Partition Chromatography" Maciuk et al., Anal. Chem. (2004) 76, 6179-6186 discloses a displacement chromatography process in which hydroxycinnamic acid isomers are separated by providing an exchanger (or retainer) substance in the stationary phase to removably retain the hydroxycinnamic acid on the stationary phase, then displacing it from the stationary phase using a displacer substance.

Numerous types of CPC apparatus are commercially available, for example from Kromaton, part of the Rousselet Robatel Group. Typically a CPC apparatus comprises a column comprising numerous (sometimes up to 1000) small chambers, sometimes called "partition cells", which are arranged circumferentially in one or more circle for rotation around a rotation axis, these partition cells being interconnected by flow channels so that a liquid may be caused to flow sequentially through them. In one known arrangement plural partition cells are etched or machined around a disk, e.g. made of a metal such as stainless steel, and plural discs are stacked along their central rotation axis to form a rotor which can be rotated around the rotation axis. Some forms of CPC apparatus having relatively few stacked discs and relatively larger volume partition cells are sometimes called centrifugal partition extractor ("CPE") apparatus. Typical CPC apparatuses are for example disclosed in US-A-6,537,452 (Kromaton), WO-A-2004/079363 (Partus) and in other scientific and commercial literature.

In the CPC process a liquid phase is introduced into the series of partition cells and channels whilst the ring(s) of partition cells is rotated about the rotation axis. This rotation causes a centrifugal force which holds this phase, the stationary phase, in place. A second liquid phase, the mobile phase, may then be caused to flow through the partition cells, and a substance dissolved in the mobile phase can thereby be caused to partition between the mobile and stationary phases in a manner analogous to the way in which a substance partitions between a mobile eluant and a stationary solid phase in column chromatography.

Liquid-liquid chromatography apparatus may be operated in either a so called "descending" or "ascending" mode. When the column is rotated, if the mobile and stationary phases have different densities the centrifugal force generated by the rotation causes the more dense phase to be radially more outward from the axis of rotation of the column than the less dense phase. If in such an apparatus the mobile phase is the more dense radially more outward phase, this is termed the "descending mode" of operation. If the opposite, i.e. the mobile phase is the less dense radially more inward phase this is termed "ascending mode".

CPC has been used for separation of various types of substances. For example separation of glucosinolates (organic compounds derived from glucose and an amino acid), is described in Toribio A, Nuzillard J-M, Renault J-H; Journal of Chromatography A. 1170 (2007) 44-51. For example purification of peptides using CPC is disclosed in WO-A-2011/157803.

It is an object of the present invention to provide a process using liquid-liquid chromatography for separation of oligonucleotides from impurities, which is both efficient in separation and is suitable for use on an industrial production scale.

According to the present invention a method for the separation of a target oligonucleotide from a mixture of the target oligonucleotide and one or more impurity using liquid-liquid chromatography is provided according to claim 1.

Thereafter the target oligonucleotide may be isolated from the second mobile phase.

In an embodiment of this method, causing the first liquid mobile phase to carry the target oligonucleotide in the flow may be achieved by providing a first liquid mobile phase containing the target oligonucleotide, e.g. having the target oligonucleotide dissolved therein.

In an embodiment of this method suitable for CPC, the method comprises the steps of:
(1) providing a first liquid phase containing the target oligonucleotide and one or more impurity in solution, and a second liquid phase containing []the exchanger substance that removably binds to the target oligonucleotide, the first and second liquid phases forming two distinct phases when in contact with each other;
(2) introducing the second liquid phase into a centrifugal partition chromatography apparatus as a stationary liquid phase therein;
(3) introducing the first liquid phase containing the target oligonucleotide and one or more impurity in solution into the centrifugal partition chromatography apparatus as a first mobile phase and causing this first liquid phase to flow through the centrifugal partition chromatography apparatus in contact with the second liquid phase such that the target oligonucleotide becomes removably bound to the exchanger substance in the second liquid stationary liquid phase;
(4) introducing a liquid phase which forms a distinct phase when in contact with the second liquid phase and which contains in solution at least one displacer substance able to displace the target oligonucleotide from the second liquid phase into the centrifugal partition chromatography apparatus, as a second mobile phase, and causing this second mobile phase to flow through the centrifugal partition chromatography apparatus in contact with the stationary liquid phase such that the target oligonucleotide becomes displaced from the stationary phase and enters solution in the second mobile phase;
(5) isolating the displaced target oligonucleotide from the second mobile phase.

Oligonucleotides typically contain 50 or less bases, and as mentioned above oligonucleotides of up to 200 bases can be easily synthesized. The method of this invention appears to be suitable for all of such oligonucleotides, and for both naturally occurring and synthetic oligonucleotides, for example oligonucleotides having base modifications or modified bases. The term "oligonucleotide" as used herein includes both DNA and RNA, LNA (Locked Nucleic Acid, i.e. in which the ribose moiety of an LNA nucleotide is modified with an extra bridge connecting the 2' oxygen and 4' carbon) sequences, oligonucleotides with 2' modifications, phosphodiesters and phosphorothioates, and includes protected and unprotected sequences.

The present invention facilitates the separation of the target oligonucleotide from impurities which for example result from the synthesis or extraction of the oligonucleotide. Separation may for example result from impurities not becoming bound to the exchanger substance in the stationary phase, so that the impurity flows through and out of the CPC apparatus still dissolved in the mobile phase. Alternately such impurities may become bound to the exchanger substance in the stationary phase but to a different extent than the target oligonucleotide, and/or may be displaced from the stationary phase but to a different extent than the oligonucleotide of interest, such that as the second mobile phase flows through the CPC the oligonucleotide of interest and the impurity(ies) separate into fractions in the stream of second mobile phase, analogous to conventional chromatography. Both of such separation processes may occur.

The process of the invention appears to be suitable for oligonucleotides of the typical lengths used in therapy, e.g. 5 - 50 bases, for example 10 - 30 bases, for example 15 - 25 bases. Presently available data suggests that process of the invention may be applied to oligonucleotides of any base sequence. The target oligonucleotide may for example be the 20 base RNA oligonucleotide designated GSK2402968 which has the sequence 5'-uca agg aag aug gca uuu ca-3'. An example of a 10 base DNA oligonucleotide is 5' GGC CAA ACC T 3'. Another example of a 20 base DNA oligonucleotide is 5' GGC CAA TCG GCT TAC CT 3'. An example of a 30 base DNA oligonucleotide is 5' GGC CAA TCG GCT TCA CTC GGC CAA ACC 3'. An example of a LNA oligonucleotide is 5' TTT ACG ACG ACG TTT 3'. An example of an siRNA oligonucleotide is 5'-gca cga uuc uca aga ugc cg-3'.

Oligonucleotides used in the process of this invention may also comprise a peptide nucleic acid or a derivative thereof.

The term "base modification" or "modified base" as identified herein refers to the modification of an existing base (i.e. pyrimidine or purine base) or to the *de novo* synthesis of a base. This *de novo* synthesized base could be qualified as "modified" by comparison to an existing base.

Other chemistries and modifications of oligonucleotides encompassed within the present invention are defined below. These additional chemistries and modifications may be present in combination with the chemistry already defined for oligonucleotides, e.g. the presence of a 5-methylcytosine, a 5-methyluracil and/or a 2,6-diaminopurine, and oligonucleotides comprising a 2'-*O*-methyl phosphorothioate RNA.

In addition to the modifications described above, oligonucleotides as used in the process of the invention may comprise further modifications such as different types of nucleic acid monomers or nucleotides as described below. For example an oligonucleotide may have at least one backbone, and/or sugar modification and/orat least one base modification compared to an RNA-based oligonucleotide.

The term base modification also includes modified versions of the natural purine and pyrimidine bases (e.g. adenine, uracil, guanine, cytosine, and thymine), such as hypoxanthine, orotic acid, agmatidine, lysidine, 2-thiopyrimidine (e.g. 2-thiouracil, 2-thiothymine), G-clamp and its derivatives, 5-substituted pyrimidine (e.g. 5-methylcytosine, 5-methyluracil, 5-halouracil, 5-propynyluracil, 5-propynylcytosine, 5-aminomethyluracil, 5-hydroxymethyluracil, 5-aminomethylcytosine, 5hydroxymethylcytosine, Super T), 2,6-diaminopurine, 7-deazaguanine, 7-deazaadenine, 7-aza-2,6-diaminopurine, 8-aza-7-deazaguanine, 8-aza-7-deazaadenine, 8-aza-7-deaza-2,6-diaminopurine, Super G, Super A, and N4-ethylcytosine, or derivatives thereof; N²-cyclopentylguanine (cPent-G), N²-cyclopentyl-2-aminopurine (cPent-AP), and N²-propyl-2-aminopurine (Pe-AP), or derivatives thereof; and degenerate or neutral bases, like 2,6-difluorotoluene or absent bases like abasic sites (e.g. 1-deoxyribose, 1,2-dideoxyribose, 1-deoxy-2-*O-*methylribose; or pyrrolidine derivatives in which the ring oxygen has been replaced with nitrogen (azaribose)). Examples of derivatives of Super A, Super G and Super T can be found in US-A-6,683,183. cPent-G, cPent-AP and Pr-AP were shown to reduce immunostimulatory effects when incorporated in siRNA (Peacock H. et al. J. Am. Chem. Soc. (2011), 133, 9200).

Oligonucleotides used in this invention may comprise an abasic site or an abasic monomer. An abasic site or monomer is a monomer or building block that lacks a nucleobase by comparison to a corresponding monomer comprising a nucleobase. An abasic monomer is thus a building block part of an oligonucleotide but lacking a nucleobase. Such an abasic monomer may be present or linked or attached or conjugated to a free terminus of an oligonucleotide. An abasic monomer may be of any type known and conceivable by the skilled person, non limiting examples of which are depicted below: 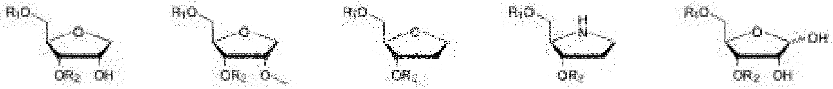 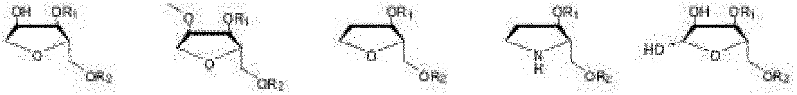 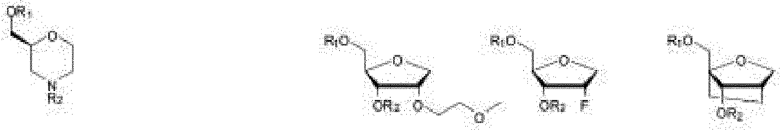 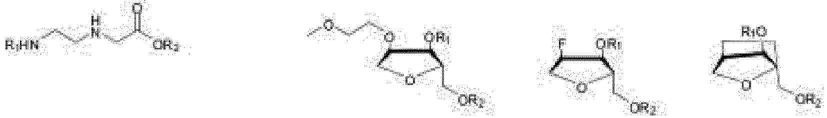 where, R₁ and R₂ are independently H, an oligonucleotide or other abasic site(s), provided that not both R₁ and R₂ are H and R₁ and R₂ are not both an oligonucleotide. An abasic monomer (s) can be attached to either or both termini of the oligonucleotide as specified before. It should be noted that an oligonucleotide attached to one or two an abasic site(s) or abasic monomer(s) may comprise less than 10 nuceotides.

Oligonucleotides used in the process of this invention may include sugar modifications i.e. a modified version of the ribosyl moiety, such as a 2'-*O*-modified RNA such as 2'-*O*-alkyl or 2'-*O*-(substituted)alkyl e.g. 2'-*O*-methyl, 2'-*O*-(2-cyanoethyl), 2'-*O*-(2-methoxy)ethyl (2'-MOE), 2'-*O*-(2-thiomethy)ethyl, 2'-*O*-butyryl, 2'-*O*-propargyl, 2'-*O*-allyl, 2'-*O*-(3-amino)propyl, 2'-*O*-(3-(dimethylamino)propyl), 2'-*O*-(2-amino)ethyl, 2'-*O*-(dimethylamino)ethyl; 2'-deoxy (DNA); 2'-*O-*(haloalkoxy)methyl (Arai K. et al. Bioorg. Med. Chem. 2011, 21, 6285) e.g. 2'-*O*-(2-chloroethoxy)methyl (MCEM), 2'-*O*-(2,2-dichloroethoxy)methyl (DCEM); 2'-*O*-[2-(N-methylcarbamoyl)ethyl] (MCE), 2'-*O*-[20(N,N-dimethylcarbamoyl)ethyl] (DCME);2'-halo e.g. 2'-F, FANA (2'-F arabinosyl nucleic acid); carbasugar and azasugar modifications; 3'-*O*-alkyl e.g. 3'-*O*-methyl, 3'-*O*-butyryl, 3'-*O*-propargyl; and their derivatives.

Other sugar modifications include "bridged" or "bicyclic" nucleic acid (BNA), e.g. locked nucleic acid (LNA), xylo-LNA, α-L-LNA, β-D-LNA, cEt (2'-*O*,4'-C constrained ethyl)LNA, cMOEt (2'-*O*,4'-*C* constrained methoxyethyl) LNA, ethylene-bridged nucleic acid (ENA), tricyclo DNA; unlocked nucleic acid (UNA); cyclohexenyl nucleic acid (CeNA), altriol nucleic acid (ANA), hexitol nucleic acid (HNA), fluorinated HNA (F-HNA), pyranosyl-RNA (p-RNA), 3'-deoxypyranosyl-DNA (p-DNA); morpholino (as e.g. in PMO, PPMO. PMOPlus, PMO-X); and their derivatives.

Oligonucleotides used in the process of this invention may include a backbone modification, e.g. a modified version of the phosphodiester present in RNA, such as phosporothioate (PS), chirally pure phosporothioate, phosporodithioate (PS2), phosponoacetate (PACE), phosponoacetamide (PACA), thiophosphonoacetate, thiophosphonoacetamide, phosphorothioate prodrug, H-phosphonate, methyl phosphonate, methyl phosphonothioate, methy phosphate, methylphosporothioate, ethyl phosphate, ethyl phosphorothioate, boranophosphate, boranophosphorothioate, methyl boranophosphate, methyl boranophosphoro thiate, methyl boranophosphonate, methyl boranophosphonothioate, and their derivatives. Another modification includes phosphoramidite, phosphoramidate, N3'→ P5' phosphoramidate, phosphordiamidate, phosphorothiodiamidate, sulfamate, dimethylenesulfoxide, sulfonate, triazole, oxalyl, carbamate, methyleneimino (MMI), and thioacetamido nucleic acid (TANA); and their derivatives.

Oligonucleotides used in the process of this invention may include other modifications, such as peptide-base nucleic acid (PNA), boron-cluster modified PNA, pyrrolidine-based oxy-peptide nucleic acid (POPNA), glycol- or glycerol-based nucleic acid (GNA), threose-based nucleic acid (TNA), acyclic threoninol-based nucleic acid (aTNA), morpholino-based oligonucleotide (PMO, PPMO. PMO-X), cationic morpholino-based oligomers (PMOplus), oligonucleotides with integrated bases and backbones (ONIBs), pyrrolidine-amide oligonucleotides (POMs); and their derivatives. A person skilled in the art will also recognize that there are many synthetic derivatives of oligonucleotides. A backbone modification includes a modified version of the phosphodiester present in RNA, such as phosphorothioate (PS), chirally pure phosphorothioate, phosphorodithioate (PS2), phosphonoacetate (PACE), phosphonoacetamide (PACA), thiophosponoacetate, thiophosphonoacetamide, phosphorothioate prodrug, H-phosphonate, methyl phosphonate, methyl phosphonothioate, methyl phosphate, methyl phosphorothioate, ethyl phosphate, ethyl phosphorothioate, boranophosphate, boranophosphorothioate, methyl boranophosphate, methyl boranophosphorothioate, methyl boranophosphonate, methyl boranophosphorothioate, and their derivatives. Another modification includes phosphoramidite, phosphoramidate, N3'→ P5' phosphoramidate, phosphordiamidate, phosphorothiodiamidate, sulfamate, dimethylsulfoxide, sulfonate, and thioacetamido nucleic acid (TANA); and their derivatives.

Each sugar, base, and/or backbone need not be modified the same way. Several distinct modified sugars, bases and/or backbones may be combined into one single oligonucleotide in oligonucleotides used in the process of this invention. Oligonucleotides used in the process of this invention may include more than one distinct base modification and/or more than one distinct sugar modification and/or one or more distinct backbone modification in said oligonucleotide.

The process of the invention appears to be suitable for separating an oligonucleotide from impurities which are not oligonucleotides.

The process also appears to be suitable for separating a target oligonucleotide from one or more impurity which is also an oligonucleotide. For example the process of the invention appears to be able to separate an oligonucleotide impurity which differs from the target oligonucleotide by only one or two bases. For example the process of the invention appears to be suitable for the separation of a target oligonucleotide having 20 bases from impurities being shortmer and longmer oligonucleotides having 17, 18, 19 or 21 bases. Such oligonucleotide impurities are believed to become bound to the exchanger substance in the stationary phase but to a different extent than the target oligonucleotide of interest, and/or may be displaced from the stationary phase but to a different extent than the target oligonucleotide of interest, such that as the second mobile phase flows through the CPC the target oligonucleotide of interest and the impurity oligonucleotides separate into fractions in the stream of second mobile phase.

In the process of the present invention the target oligonucleotide may be as the free oligonucleotide or may be in the form of a derivative of a target oligonucleotide, such as in a form protected by a protecting group. Numerous protecting groups for oligonucleotides are known. A suitable protecting group is dimethoxy trityl, and it is believed that other known protecting groups are also suitable.

The first mobile phase and stationary phase may comprise any liquids which form two distinct phases when in contact with each other, and which comprise a mobile phase which dissolves the target oligonucleotide, and a stationary phase which dissolves the exchanger substance and the target oligonucleotide when bound to the exchanger substance. Other desirable properties of such liquids include the formation of two phases with a difference in density that they easily form two distinct phases and show little tendency to emulsify.

For example these two phases may comprise combinations of solvents or of water and one or more solvent which when mixed produce a biphasic system in which the exchanger substance is only or substantially soluble in only one of the (mobile or stationary) phases, and the displacer substance is only or substantially soluble in only the other (stationary or mobile) phase.

For example the stationary phase may comprise a mixture of one or more organic liquid which is immiscible or partially miscible with water such as a C₁₋₆ alkyl C₁₋₆alkanoate ester, a di- C₁₋₆ alkyl ether, a C₄₋₁₀ cyclic ether, a liquid halogenated C₁₋₆ alkane, or a liquid C₅₋₁₀ alkane; and one or more organic liquid which is at least partially miscible with water such as a C₁₋₈ alkanol or di-(C₁₋₈ alkyl) ketone.

By "partially miscible" is included that a two-phase system can be formed over at least part of the alkanol-water composition range. Each of such two phases contains both alkanol and water, but in one phase alkanol will predominate and in the other phase water will predominate. Lower, e.g. C₁₋₃ alkanols are generally fully miscible with water over the entire water-alkanol composition range, but higher e.g. C₄₋₈ alkanols are generally immiscible or only partially miscible with water. For example the first and second mobile phases may comprise a mixture of one or more organic liquid which is miscible with water such as a C₁₋₈ alkanol or di-(C₁₋₈ alkyl) ketone; and water. Examples of such organic liquids include ethyl acetate, methyl-tertbutyl ether, alkyl-substituted tetrahydrofurans such as 2-methyltetrahydrofuran, chloroform, hexane, 1-butanol, ethanol, methanol and highly polar aprotic solvents such as dimethyl sulphoxide and dimethyl formamide.

It will of course be appreciated that in any such equilibrated system in which such two phases are in contact with each other there may be some mutual intermixing of minor proportions of the liquid components such that the stationary phase liquid may contain a minor proportion of water dissolved in from the mobile phase liquid, and the mobile phase liquid may contain a minor proportion of the liquid which is substantially immiscible with water dissolved in from the stationary phase liquid.

Suitably the respective phases may be formed by mixing two or more of such liquids and allowing the system to settle into two equilibrium phases being an upper less dense and lower more dense phase each of which may be used as a stationary or mobile phase. The stationary and mobile phases may therefore comprise the respective two equilibrium phases of a liquid system containing such liquids. It will be appreciated that each of such phases may contain all of the liquids comprising the system, but each such phase will contain predominantly one or more of the liquids as major component, and one or more of the liquids as minor component.

Examples of such phases include two phase systems (a) to (e) listed below. The relative proportions in which such liquids may be mixed to provide such phases will depend upon the liquids but may easily be determined experimentally. Typical proportions are suggested below.
(a) C₁₋₆ alkyl C₁₋₆ alkanoate ester - C₁₋₈ alkanol-water. Less dense phase comprises major component ester + alkanol. More dense phase comprises major component water + alkanol. For example ethyl acetate -1-butanol-water (3 : 2 : 5).
(b) C₄₋₈ alkanol-water. Less dense phase comprises major component alkanol. More dense phase comprises major component water. For example 1-pentanol or 1-butanol - water.
(c) C₁₋₈ alkanol - di-(C₁₋₈ alkyl) ketone - water. Less dense phase comprises major component alkanol + ketone. More dense phase comprises major component water + alkanol. For example 1-butanol-methylisobutyl ketone-water (1 : 3 : 4).
(d) di-(C₁₋₈ alkyl) ketone - water. Less dense phase comprises major component ketone. More dense phase comprises major component water. For example methylisobutyl ketone - water.
(e) di-C₁₋₆ alkyl ether or a C₄₋₁₀ cyclic ether - C₁₋₈ alkanol - water. Less dense phase comprises major component ether and alkanol. More dense phase comprises major component water. For example methyl-tertiarybutylether-1-pentanol-water (3 : 1 : 4) or 2-methyltetrahydrofuran-1-butanol-water (3 : 1 : 4).

It will be understood that in addition to its own major components each of such phases in a pair will contain minor component(s) being the major component of the other phase in the pair. In the process of this invention the stationary phase may be the less dense of such pairs of phases.

A preferred first mobile phase comprises a mixture of water and 1-butanol as major components, with a stationary phase which comprises a mixture of ethyl acetate and 1-butanol as major components. Another preferred first mobile phases comprises a mixture of water and 1-butanol as major components, with a stationary phase which comprises a mixture of 2-methyltetrahydrofuran and 1-butanol as major components. Such mixtures form two distinct phases when in contact with each other.

Suitably the first mobile phase may be adjusted to a suitable pH to facilitate dissolution and stabilization of the target oligonucleotide, for example by the inclusion of a base, for example an inorganic base such as an alkali metal hydroxide such as sodium hydroxide, or ammonia. A suitable pH depends inter alia on the oligonucleotide and may depend on whether it is protected. A suitable pH range for the first mobile phase appears to be pH7-14, although it is believed acid pH <7 may be feasible for some applications. A suitable pH range for the above-mentioned 20-mer oligonucleotide in either dimethoxy trityl-protected form or unprotected is ca. pH 7-12. A suitable pH range for this oligonucleotide in dimethoxy trityl-protected form is e.g. pH 10-12, suitably around pH 11. Presently available data suggests such pH ranges may be suitable for other oligonucleotides. A suitable concentration for such a base in the first mobile phase to achieve such a pH may be determined experimentally. In the experiments described herein a suitable concentration was found to be 10 +/-5 mM, for example 10 mM, sodium hydroxide.

A suitable concentration of the target oligonucleotide, such as the 20-mer referred to above, may be determined experimentally. A concentration in the column of 200mg - 100g per L, for example 200mg - 80g per L, for example 20-60 g per L appears to be suitable.

The exchanger substance (sometimes in the art alternatively referred to as the "retainer") removably binds to the target oligonucleotide in the stationary phase. Suitably the exchanger substance is an anion-exchanger substance.

The exchanger substance which binds to a target oligonucleotide is a secondary, tertiary or quaternary ammonium salt which has the general formula:

R¹R²R³R⁴N⁺ X⁻

wherein through the sequence secondary, tertiary and quaternary respectively two, three or four of the groups R¹, R², R³ and R⁴ are independently C₁₋₂₀ alkyl or substituted alkyl such as fluoro or trifluoromethyl substituted alkyl, or benzyl and the remainder are hydrogen, and X- is a halide anion suitably chloride. A suitable exchanger substance is a mixture of tri-(n-octyl) methyl ammonium chloride and tri-(n-decyl) methyl ammonium chloride, suitably in which the n-octyl compound predominates. Such a mixture is commercially available under the name Aliquat 336™. Other exchanger substances believed to be suitable include cetyltrimethylammonium bromide, methyltrioctylammonium chloride, benzalkonium chloride (also known as alkyldimethylbenzylammonium chloride and ADBAC, being a mixture of alkylbenzyldimethylammonium chlorides of various alkyl chain lengths), benzyltrimethylammonium chloride, tetrabutylammonium chloride, and Amberlite LA2 (a high molecular weight, oil soluble secondary amine supplied as a liquid in the free-base form) in protonated form.

The concentration of the exchanger substance in the stationary phase will depend inter alia upon the substance used, the target oligonucleotide itself, and the flow rate of the mobile phase. A calculation can be made on the basis of molar ratios of the target oligonucleotide and the exchanger substance. For a 10 to 30mer oligonucleotide a suitable range of molar ratio of the exchanger substance is believed to be 0.5 - 15, preferably ca. 5, equivalents per ionic site, leading to a molar ratio exchanger substance : target oligonucleotide in the range 1 : 5 - 500, preferably 1 : 10 - 300, more preferably 1: 20-150. A concentration of exchanger substance of 5 - 500, for example 5 - 100, typically 8-50 mM appears to be suitable, at least for Aliquat 336™, and possibly for other exchanger substances.

It is believed to be possible to use any commercially available liquid-liquid chromatography apparatus that uses liquid - liquid stationary and mobile phase systems to perform the method of this invention.

An example of a suitable CPC apparatus is a FCPC200™ CPC apparatus from Kromaton. Suitable CPC instruments are also available from Armen Instrument. The normal specification operating conditions for such apparatus appear to be suitable to perform the process of the present invention. The stationary phase is suitably introduced into the column following the normal operating procedure for the apparatus.

In typical operation the column may be filled with stationary phase by pumping the stationary phase liquid, e.g. the second liquid phase, at a high flow rate into the column, then starting rotation of the column at a suitable operating speed for the apparatus.

The first mobile phase may be caused to carry the target oligonucleotide (together with impurities) in its flow in various ways.

In one way for example first mobile phase containing the dissolved target oligonucleotide may then be introduced into the column and caused to flow through the column relative to and in contact with the stationary phase.

In another way for example the target oligonucleotide may be dissolved in a liquid which is miscible with the mobile phase, for example which is a liquid component of the first mobile phase liquid, or contains one or more liquid component of the mobile phase, and introduced in this form into a flow of the first mobile phase. If the first mobile phase liquid contains water the target oligonucleotide may for example be introduced in this way as an aqueous solution. Suitably with a water-containing first mobile phase the target oligonucleotide, typically in its crude state combined with contaminating impurities, may be mixed in the form of an aqueous solution, e.g. in aqueous ammonia, with a water-containing first mobile phase. Some syntheses of oligonucleotides produce the oligonucleotide in solution in a mixture of water, dissolved ammonia, and solvents such as acetonitrile and ethanol, and in some applications the target oligonucleotide may be introduced into the column in the form of such a solution. In this way of introducing the target oligonucleotide the liquid which is miscible with the mobile phase may become mixed with the mobile phase during its flow, or may remain as a substantially distinct phase and be pushed around the column by the mobile phase.

In an optional step, prior to the introduction of first mobile phase containing the dissolved target oligonucleotide into the column, first mobile phase free of the target oligonucleotide may be caused to flow through the column filled with stationary phase. Such an initial flow of mobile phase can elute some stationary phase, which continues until hydrodynamic equilibrium is reached and the mobile phase leaving the exit end of the column contains no or minimal stationary phase. Such a state can be measured by sampling the flow from the exit end of the column.

Optionally the first mobile phase containing the dissolved target oligonucleotide may be introduced into the column as a mixture with stationary phase liquid. Introducing the mobile phase as such a mixture with stationary phase liquid may help to ensure that the mobile phase remains saturated with the stationary phase, and can prevent stripping of the stationary phase from the column by the mobile phase as it flows through the column. The quantity of stationary phase needed if this is done can be determined experimentally, and may need to be a few percent.

Suitable flow rates for the stationary and mobile phases may be determined by the normal operating procedure for the liquid-liquid chromatography apparatus, such as the Armen or Kromaton, e.g. Kromaton FCPC200™. Introduction of the first mobile phase can have the effect over a brief time of displacing a quantity of stationary phase, which can be detected. Since the volume of stationary phase and the column volume are known, it is possible to calculate Sf, the stationary phase retention factor, being a measure of the proportion of stationary phase remaining in the column.

The flow of the mobile phase containing the target oligonucleotide through the column containing the stationary phase containing the exchanger substance causes the target oligonucleotide dissolved in the first mobile phase to become bound to the exchanger substance in the stationary phase. The ability of the target oligonucleotide to be retained in the stationary phase depends inter alia on the time allowed for equilibration and the concentration of the exchanger substance in the stationary phase. Suitable flow rates, for example suitable flow rate of the first mobile phase containing the dissolved target oligonucleotide into the column to achieve an equilibrium in which all or a suitable proportion of the target oligonucleotide is retained in the stationary phase can be determined experimentally based upon the operating conditions of the instrument. A suitable flow rate, at least for the Kromaton FCPC200™ and possibly generally, appears to be 2-6 ml per minute, but is expected that such instruments may be run faster. Different flow rates may be appropriate to other instruments, particularly for larger scale instruments. Suitable conditions may be determined experimentally, for example by monitoring the concentration of the target oligonucleotide, and/or other substances, in the stream of first mobile phase exiting from the CPC apparatus. Suitable monitoring techniques will be apparent to those skilled in the art, for example using spectroscopy or HPLC etc.

The second liquid mobile phase may have the same composition of liquids, e.g. one or more solvent or a mixture of one or more solvent and water, as the first mobile phase, e.g. as described above. For example as described above in combination with a stationary phase comprising a mixture of predominantly C₁₋₈ alkanol such as 1-butanol and C₁₋₆ alkyl C₁₋₆ alkanoate ester such as ethyl acetate the second mobile phase may comprise a mixture of water and a C₁₋₈ alkanol such as 1-butanol, which if provided in the manner described above may also contain some C₁₋₆ alkyl C₁₋₆ alkanoate ester in a minor proportion. Alternatively for example as described above in combination with a stationary phase comprising a mixture of predominantly C₁₋₆ cyclic ether such as methyltetrahydrofuran and C₁₋₈ alkanol, the second mobile phase may comprise a mixture of predominantly water and the C₁₋₈ alkanol.

Suitably the second mobile phase may also be adjusted to a suitable pH to facilitate dissolution and stabilization of the target oligonucleotide. A suitable pH depends inter alia on the oligonucleotide and may depend on whether it is protected. A suitable pH range for the above-mentioned 20-mer oligonucleotide in either dimethoxy trityl-protected form or unprotected is ca. pH 7-12. A suitable pH range for this oligonucleotide in dimethoxy trityl-protected form is e.g. pH 10-12, suitably around pH 11. Such pH ranges, e.g. pH 7-14, may be suitable for other oligonucleotides, and it is believed that for some applications acid pH <7 may be feasible. Adjustment of pH may for example be by addition of a base such as sodium hydroxide. A suitable concentration for such a base in the second mobile phase to achieve such a pH is 10 +/-5 mM, for example 10 mM sodium hydroxide.

Suitable displacer substances are substances which have a greater binding constant, e.g. ability to form an electrostatic bond, with the exchanger substance than the target oligonucleotide. Suitable displacer substances incorporate an anionic moiety, which may be singly or multiple charged. Suitable displacer substances include salts, especially salts of inorganic acids such as halides, sulphates etc. or oxalates, with metals especially alkali metals. A suitable displacer substance is sodium or potassium iodide. Other suitable displacer substances include saccharin in its deprotonated form such as alkali metal salts of saccharin such as saccharin sodium salt, Sunset Yellow (Disodium 6-hydroxy-5-[(4-sulfophenyl)azo]-2-naphthalenesulfonate) and Amaranth (trisodium (4E)-3-oxo-4-[(4-sulfonato-1-naphthyl) hydrazono] naphthalene -2,7-disulfonate). It will be appreciated that the latter two incorporate di- and tri-basic anionic moieties.

A calculation can be made on the basis of molar ratios of exchanger substance : displacer substance, which is preferably in the range 1 : 1 to 5 : 1. On this basis a maximum concentration for the displacer substance appears to be ca. 500mM. A suitable concentration for the displacer substance in the second mobile phase appears to be 2-500 mM, for example 5-30 mM.

A suitable flow rate of the second mobile phase containing the displacer substance to achieve displacement of the target oligonucleotide from the stationary phase at a rate such that the target oligonucleotide appears as a conveniently discrete fraction in the flow of second mobile phase exiting the CPC apparatus may be determined experimentally. A suitable flow rate, at least for the Kromaton FCPC200™ was found in the experiments described herein to be 2-6 ml per minute, which appears to be able to achieve complete displacement of the target oligonucleotide, but it is believed that such apparatus can be made to run faster, for example up to 20ml per minute in a 200ml column. Suitable conditions for achieving such displacement may be determined experimentally, for example by monitoring the concentration of the target oligonucleotide in the stream of second mobile phase exiting from the CPC apparatus. Suitable monitoring techniques will be apparent to those skilled in the art, for example using spectroscopy or HPLC etc.

The target oligonucleotide may then be isolated from the second mobile phase by a conventional process, optionally involving removal of any protecting group(s), traces of exchanger and/or displacer substances etc. if necessary. Typical isolation techniques include deprotection (if the oligonucleotide is protected), desalting (ultrafiltration) and lyophilisation. The isolated target oligonucleotide may then be subjected to any further purification steps, e.g. ion-exchange chromatography, that may be considered necessary.

The present invention will now be described by way of example only with reference to the following drawings.
Fig. 1 shows a UV chromatogram of the second mobile phase exiting from the column from Experiment 8.
Fig. 2 shows a Chromatogram reconstruction after fractions analysis from Experiment 8.
Fig. 3 shows a Chromatogram reconstruction after fractions analysis from Experiment 21.
Fig. 4 shows a UV chromatogram of the second mobile phase exiting from the column from Experiment 4.
Fig. 5 shows a Chromatogram reconstruction after fractions analysis from Experiment 18.

A number of experiments were performed as set out in the table below. Two experiments listed in the table as Experiments 8 and 21 are described in detail below.

### Experiment 8.

The liquid phases were prepared according to the following procedure. Ethyl acetate, 1-butanol and water were mixed in volume ratios 3 : 2 : 5. The bi-phasic system was left to settle until two clear phases were obtained, then these phases were separated.

To the top phase layer (ethyl acetate / butanol with a minor proportion of water), was added Aliquat 336™ (exchanger substance) to obtain a concentration of 40mM and the container was labeled as "stationary phase", i.e. the second liquid phase.

The bottom phase (water/butanol with a minor proportion of ethyl acetate), was divided into two equal portions. To the first portion was added sodium hydroxide to obtain a concentration of 10mM, and the container was labeled as "first mobile phase", i.e. the first liquid phase.

To the second portion of bottom phase was added sodium hydroxide to obtain a concentration of 10mM and potassium iodide (displacer substance) until a concentration of 13.3 mM was reached, and the container was labeled "second mobile phase", i.e. the third liquid phase.

The Centrifugal Partition Chromatography (CPC) apparatus used was a commercially available 200ml Armen CPC instrument. Typically such apparatus comprise a rotor made of ca. 20 circular partition discs. Typically such apparatus can be adjusted from 200 - 2000 rpm, generating a centrifugal force of ca. 120g at 1000 rpm and 480g at 2000 rpm). The Kromaton FCPC200 apparatus was set on descending mode. The column was rotated at 1200rpm. The column was filled with the solution from the "stationary phase" container (second liquid phase plus Aliquat 336™) at a flow rate of 10mL/min., following the specification operating conditions for the apparatus. Typically commercially available Dionex P580HPG 4-way binary high pressure gradient pumps (Sunnyvale, CA, USA) may be used to introduce the stationary and mobile phases into the apparatus, typically via low-pressure injection valves (such as Upchurch, CIL, Cluxeau, Saint-Foy-La-Grande, FR), typically equipped with a 21 ml sample loop.

A sample of protected target oligonucleotide, being a 20-mer RNA oligonucleotide 5'-uca agg aag aug gca uuu ca-3', but known to be contaminated with impurities possibly including 17-, 18-, 19- and/or 21- mer impurities (400mg) was dissolved in the first mobile phase, (10mL). Once dissolved, 10mL of the stationary phase liquid (but containing no exchanger substance) was added to this solution of the oligonucleotide in the mobile phase to form a two phase mixture.

The mixture of the solution of target oligonucleotide dissolved in the first mobile phase and the stationary phase liquid was injected into the column, and this mixture was pumped through the column at a flow rate of 5mL/min for 20min. At this time equilibration had been reached, i.e. no more stationary phase was being stripped out of the column by the flowing mobile phase, and no target oligonucleotide was coming out of the column, as detected by analyzing the eluent exiting from the column using HPLC.

Then the second mobile phase (third liquid phase) was pumped at a similar flow rate for 100min. Fractions of exiting second mobile phase were collected every minute and analysed off-line.

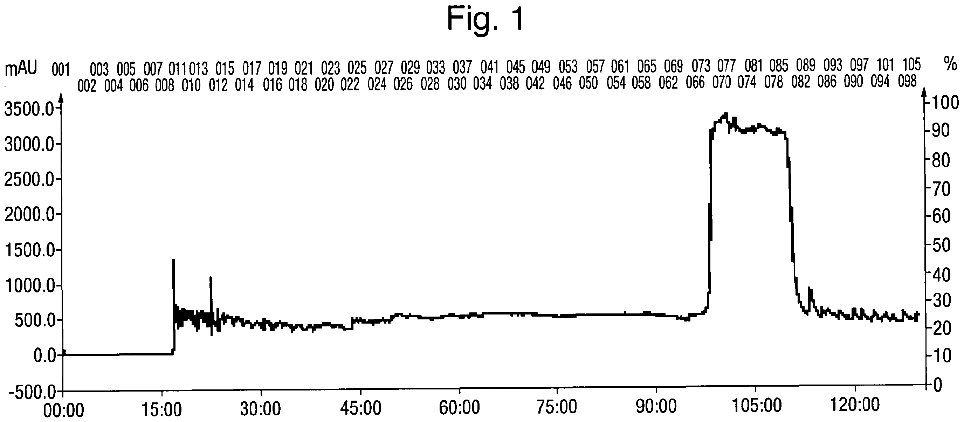

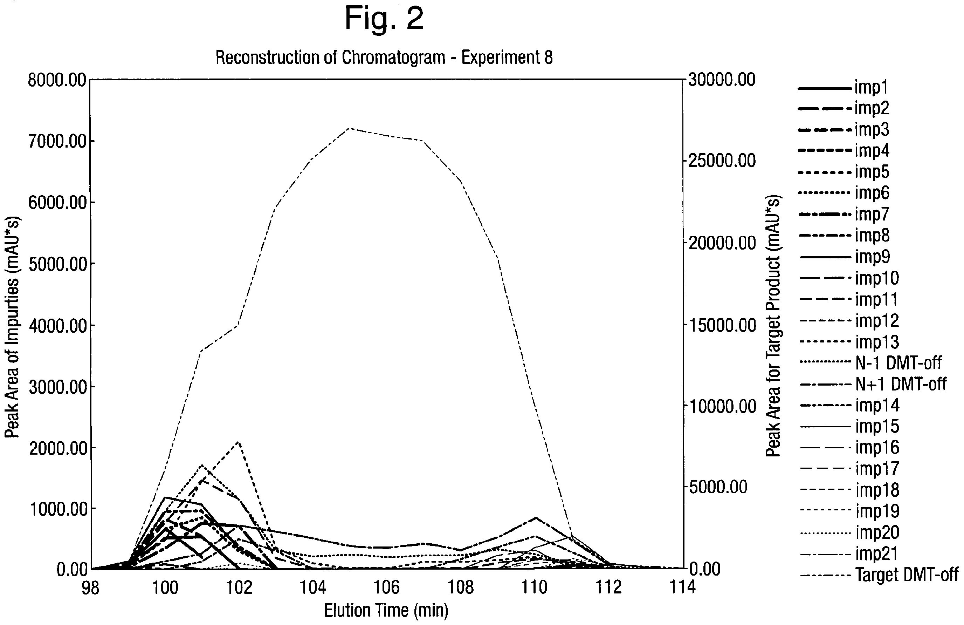

Referring to Fig. 1, this follows the introduction of the first and second mobile phases into the column starting at time = zero. During the first ca. 17 minutes a small quantity of stationary phase was displaced from the column. At ca. 20 minutes equilibrium was reached and very little stationary phase was displaced by the flowing first mobile phase. The second mobile liquid phase containing displacer substance was introduced after 20 minutes. Thereafter the peak represents elution of substances displaced by the second mobile phase from the stationary phase. Analysis of fractions from this peak indicated that the impurities present in the introduced oligonucleotide were tightly stacked at the beginning and end of this peak. Fig. 2 shows a Chromatogram reconstruction after fractions analysis from Experiment 8.

### Experiment 21.

The solvent system was composed of Me-THF/n-BuOH / Water in volume ratios (3:1:4) respectively. These three liquids were mixed and allowed to settle into two phases, which were separated.

To the upper organic phase was added Aliquat 336 (exchanger substance) to obtain a concentration of 40mM, and this organic phase was designated for use as the stationary phase.

The lower aqueous phase was divided into two portions. To one portion was added sodium hydroxide to produce a concentration of 10mM, and this portion was designated as the first mobile phase. To the second portion of the lower phase was added sodium hydroxide to produce a concentration of 10mM, and Amaranth (displacer substance) to produce a concentration of 4.4mM. This portion was designated the second mobile phase.

The crude sample of oligonucleotide (400mg containing approximately 88% of target oligonucleotide in 35 mL of aqueous ammonia) was combined with 5mL of the portion of upper phase of the solvent system designated as the stationary phase (containing no exchanger substance).

The column was rotated at 1200rpm and filled with stationary phase in descending mode. Then the sample as prepared above was loaded onto the column while pumping mobile phase 1 at 5mL/min. The first mobile phase was pumped for 10minutes, followed by the second mobile phase also at a rate of 5mL/min for 120min. Fractions were collected every minute.

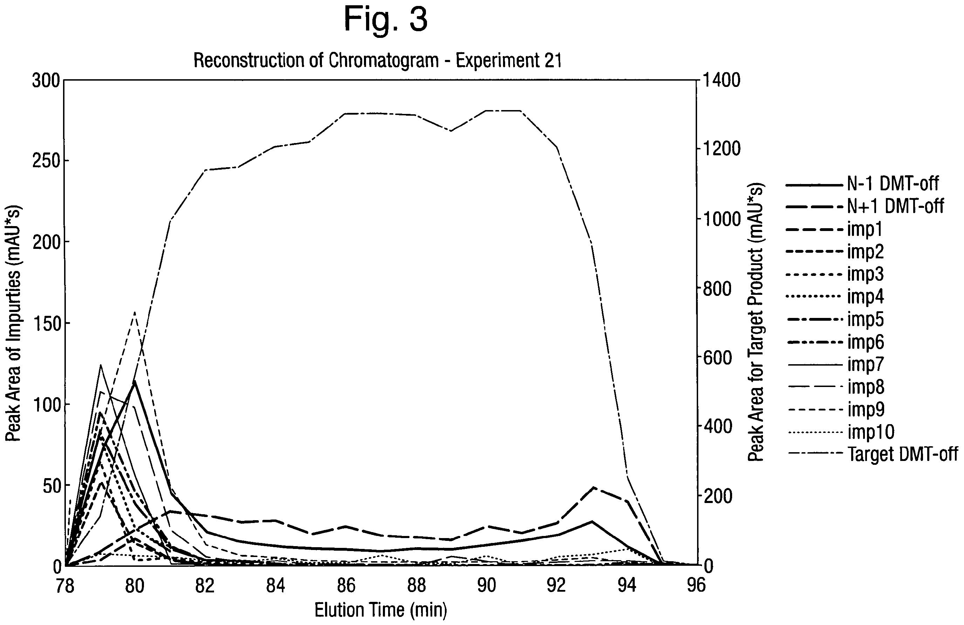

Fig. 3 shows the HPLC peaks of the eluate from Experiment 21, indicating "N DMT-off" as the target nucleotide without its DMT protecting group, "N+1" and "N-1" as the target nucleotide +/- one base also without the DMT protecting group, and imp 1-10 as traces corresponding respectively to ten impurities. It is seen that elution of impurities imp 1-10 and N-1 occurs virtually together and is largely complete before purified target oligonucleotide is eluted by the second mobile phase, and during the time period from ca. 81 to 95 minutes after introduction of the second mobile phase >95% pure target oligonucleotide is eluted. An N+1 impurity eluted mainly toward the end of the target peak.

### Further Experiments.

Details of experiments 1-30 are presented in tabular form below. Experiment 1 was a control in which no exchanger or retainer substance was used. In experiment 12 it is believed no stationary phase was retained because the two component system 2-butanol-water did not form two phases with a difference in density such that they easily formed two distinct phases with little tendency to emulsify.

In the column "oligo type" P=O refers to phosphodiester forms of the oligonucleotide, and P=S refers to phosphorothioateforms of the oligonucleotide. Al336 denotes Aliquat 336 and BACI denoted Benzalkoniun chloride. In presenting the data from these experiments a target purity was set and the amount of target material recovered with that purity was calculated. Isotachic train refers to the time in minutes during which the target oligonucleotide is eluted by the second mobile phase at the set purity. "MIBK" is an abbreviation for methylisobutylketone. "MtBE" is an abbreviation for methyltertiarybutyl ether. "MeTHF" is an abbreviation for 2-methyltetrahydrofuran.

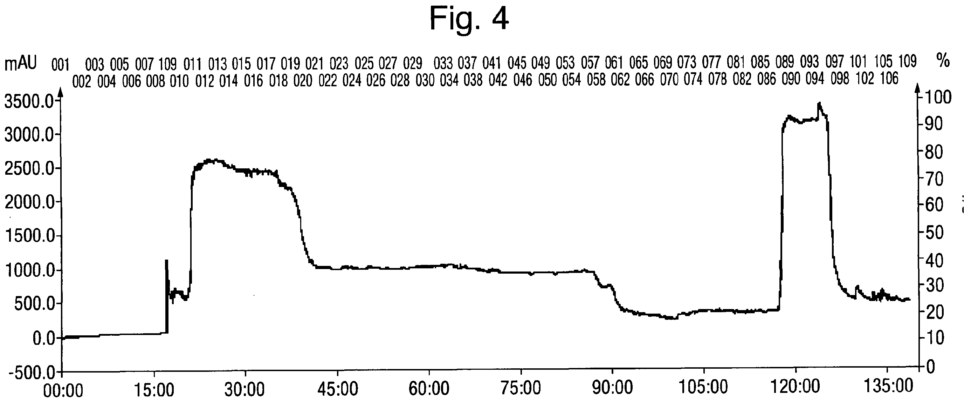

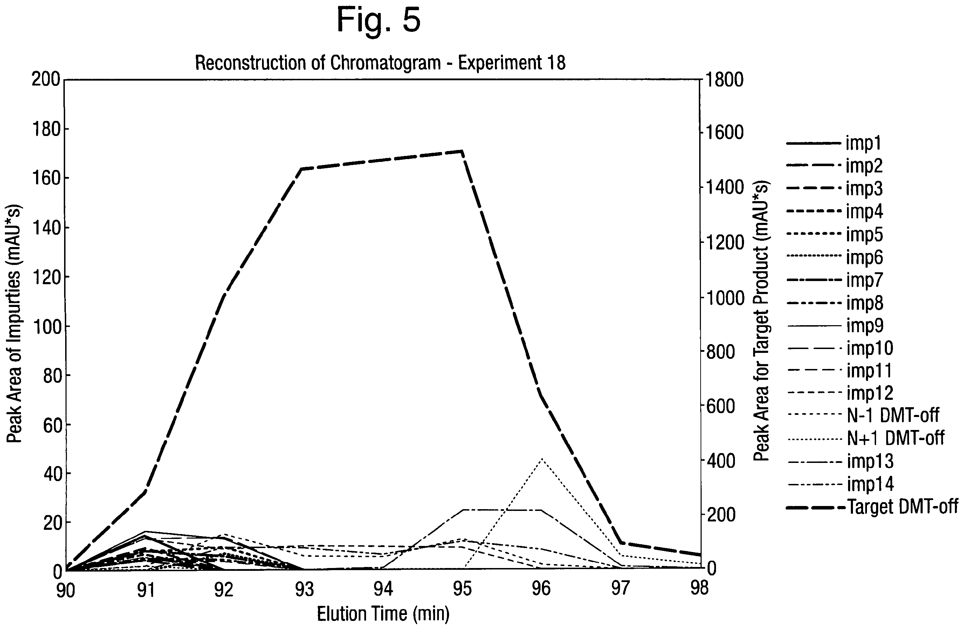

Fig. 4 shows a UV chromatogram of the second mobile phase exiting from the column from Experiment 3, and Fig. 5 shows a Chromatogram reconstruction after fractions analysis from Experiment 18. In each of these the envelope representing the target oligonucleotide shows that for substantial periods of flow of the second mobile phase, considerably purified target oligonucleotide is being eluted and can be isolated from the second mobile phase using conventional techniques.

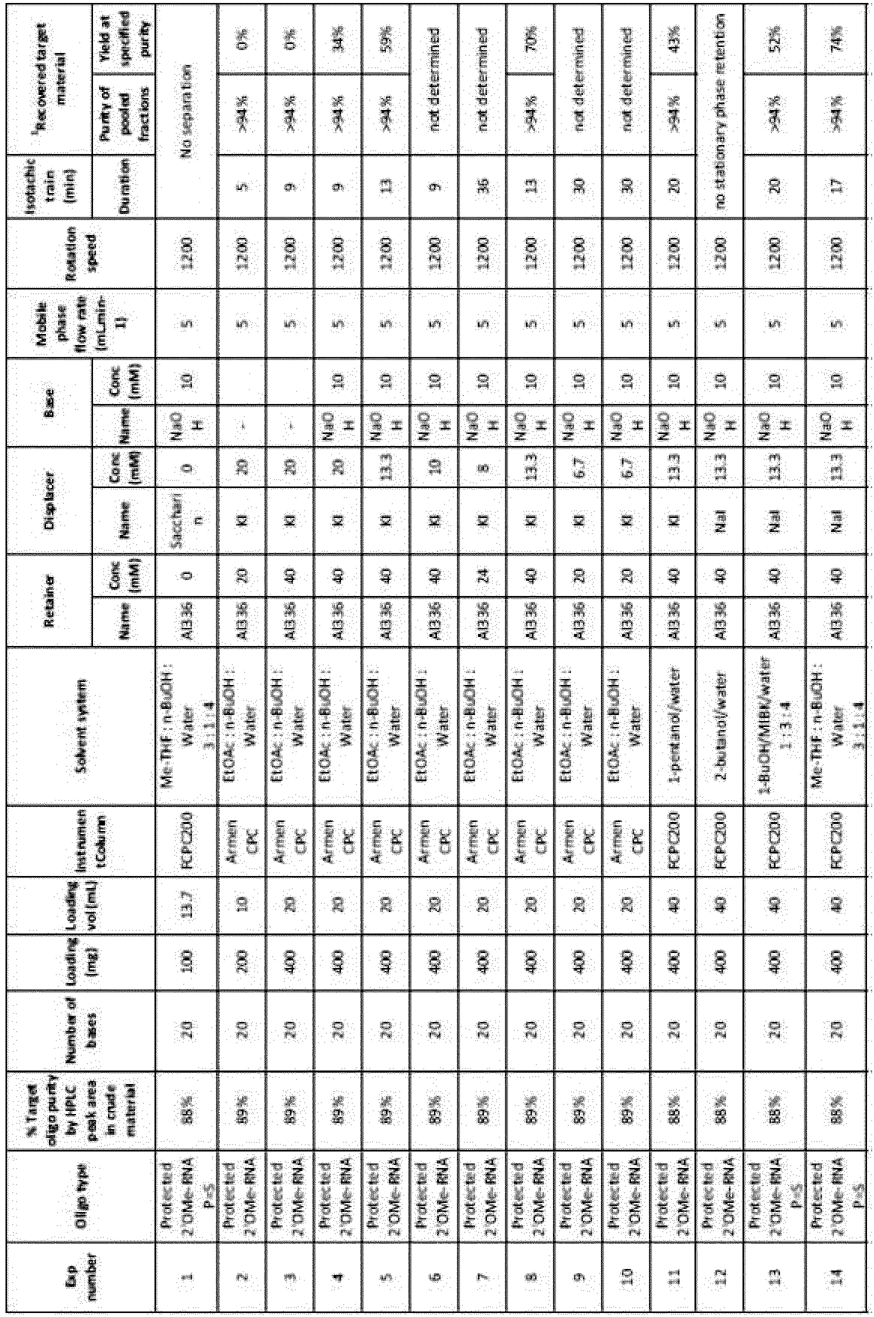

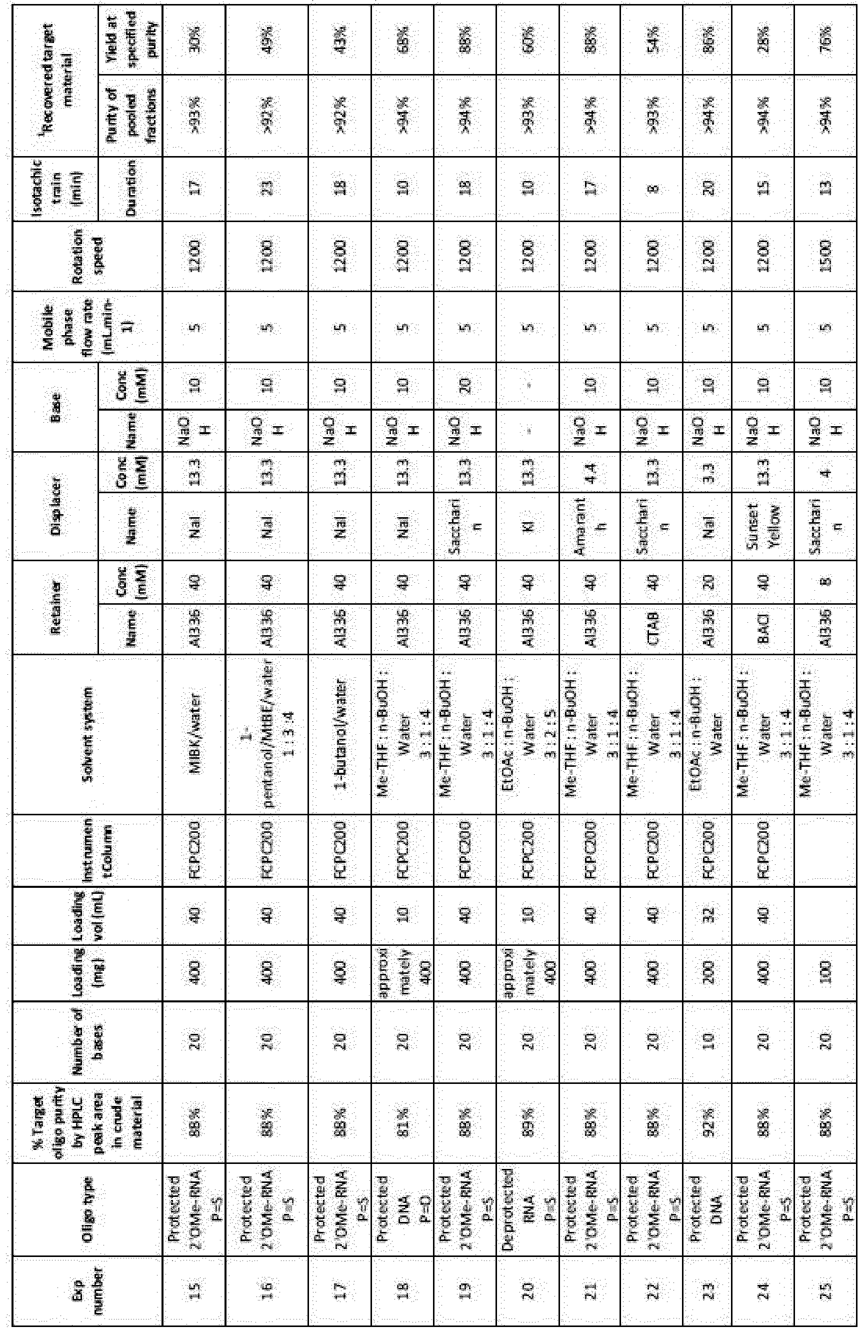

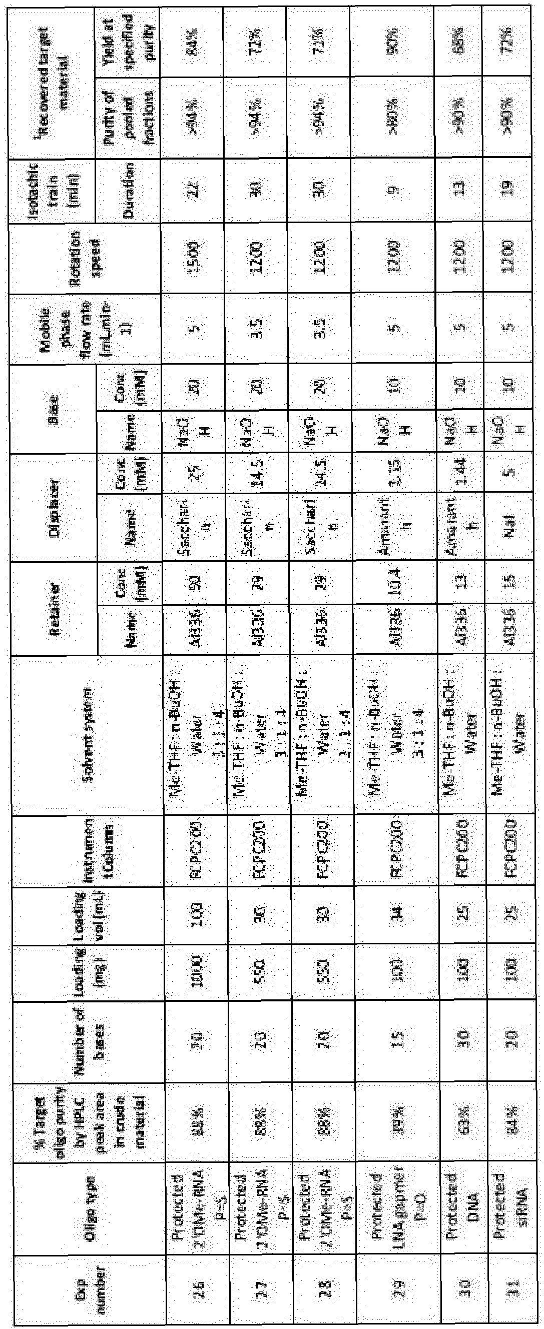

## Claims

1. A method for the separation of a target oligonucleotide from a mixture of the target oligonucleotide and one or more impurity comprising:
providing a biphasic mobile phase - stationary phase liquid - liquid chromatography system comprising a first liquid mobile phase, and a liquid stationary phase containing at least one exchanger substance that removably binds to the target oligonucleotide;
causing the first liquid mobile phase to carry the target oligonucleotide in a flow relative to and in contact with the liquid stationary phase in the column of a liquid - liquid chromatography apparatus such that the target oligonucleotide becomes bound to the exchanger substance in the liquid stationary phase;
then displacing the target oligonucleotide from the liquid stationary phase into a second liquid mobile phase flowing relative to and in contact with the stationary phase through the column by means of a displacer substance able to displace the target oligonucleotide from the liquid stationary phase into the second mobile phase,
***characterized* in that** the exchanger substance is a salt of an organic secondary, tertiary or quaternary amine with a counter anion, of the general formula:
R¹R²R³R⁴N⁺X⁻
wherein through the sequence secondary, tertiary and quaternary respectively two, three or four of the groups R¹, R², R³ and R⁴ are independently C₁₋₂₀ alkyl or substituted alkyl such as fluoro or trifluoromethyl substituted alkyl, or benzyl and the remainder are hydrogen, and X⁻ is a halide anion.

2. A method according to claim 1 ***characterised* by** the steps of:
(1) providing a first liquid phase containing the target oligonucleotide and one or more impurity in solution, and a second liquid phase containing the exchanger substance that removably binds to the target oligonucleotide, the first and second liquid phases forming two distinct phases when in contact with each other;
(2) introducing the second liquid phase into a centrifugal partition chromatography apparatus as a stationary liquid phase therein;
(3) introducing the first liquid phase containing the target oligonucleotide and one or more impurity in solution into the centrifugal partition chromatography apparatus as a first mobile phase and causing this first liquid phase to flow through the centrifugal partition chromatography apparatus in contact with the second liquid stationary phase such that the target oligonucleotide becomes removably bound to the exchanger substance in the second liquid stationary phase;
(4) introducing a third liquid phase which forms a distinct phase when in contact with the second liquid phase and which contains in solution at least one displacer substance able to displace the target oligonucleotide from the second liquid phase into the centrifugal partition chromatography apparatus, as a second mobile phase and causing this second mobile phase to flow through the centrifugal partition chromatography apparatus in contact with the second liquid phase such that the target oligonucleotide becomes displaced from the stationary phase and enters solution in the second mobile phase;
(5) isolating the displaced target oligonucleotide from the second mobile phase.

3. A method according to claim 1 or 2 ***characterised* in that** the target oligonucleotide comprises 10-30 bases.

4. A method according to claim 3 ***characterised* in that** the target oligonucleotide is the 20 base oligonucleotide which has the sequence 5'-UCAAGGAAGAUGGCAUUUCA-3'.

5. A method according to any one of the preceding claims ***characterised* in that** the stationary phase comprises a mixture of one or more organic liquid which is substantially immiscible with water and one or more organic liquid which is miscible with water.

6. A method according to claim 5 ***characterised* in that** the first and second mobile phases comprise a mixture of one or more organic liquid which is miscible with water, and water.

7. A method according to claim 5 or 6 ***characterised* in that** the stationary and mobile phases comprise the respective two equilibrium phases of: (i) a liquid system containing C₁₋₆ alkyl C₁₋₆ alkanoate ester, C₁₋₈ alkanol and water; or (ii) a liquid system containing C₁₋₈ alkanol and water; or (iii) a liquid system containing C₁₋₈ alkanol, di-(C₁₋₈ alkyl) ketone and water; or (iv) a liquid system containing di-(C₁₋₈ alkyl) ketone and water; or (v) a liquid system containing C₁₋₆ alkyl C₁₋₆ alkyl ether or a C₄₋₁₀ cyclic ether, C₁₋₈ alkanol and water.

8. A method according to any one of the preceding claims ***characterised* in that** the halide anion of the exchanger substance is chloride.

9. A method according to claim 8 ***characterised* in that** the exchanger substance is selected from a mixture of tri-(n-octyl) methyl ammonium chloride and tri-(n-decyl) methyl ammonium chloride; and benzalkonium chloride.

10. A method according to any one of claims 1 to 7 ***characterised* in that** the exchanger substance is selected from cetyltrimethylammonium bromide, methyltrioctylammonium chloride, a mixture of alkylbenzyldimethylammonium chlorides of various alkyl chain lengths, benzyltrimethylammonium chloride, tetrabutylammonium chloride, and a high molecular weight, oil soluble secondary amine supplied as a liquid in the free-base form in protonated form.

11. A method according to any one of the preceding claims ***characterised* in that** the second liquid mobile phase has the same composition of liquids as the first mobile phase.

12. A method according to claim 11 ***characterised* in that** the stationary phase comprises a mixture of predominantly C₁₋₈ alkanol and C₁₋₆ alkyl C₁₋₆ alkanoate ester, and the second mobile phase comprises a mixture of water and C₁₋₈ alkanol.

13. A method according to claim 11 ***characterised* in that** the stationary phase comprises a mixture of predominantly C₁₋₆ cyclic ether and C₁₋₈ alkanol, and the second mobile phase comprises a mixture of predominantly water and C₁₋₈ alkanol.

14. A method according to any one of the preceding claims ***characterised* in that** the displacer substance is selected from alkali metal halides, sulphates and oxalates, Saccharin sodium salt, Sunset Yellow and Amaranth.

15. A method according to any one of the preceding claims ***characterised* in that** the concentration of the displacer substance in the second mobile phase is 5-30 mM.

## Patentansprüche

1. Verfahren zur Abtrennung eines Ziel-Oligonukleotids von einer Mischung aus dem Ziel-Oligonukleotid und einer oder mehrerer Verunreinigung(en), umfassend:
das Bereitstellen eines biphasischen mobile Phase - stationäre Phase Flüssigkeits - Flüssigkeitschromatographiesystems umfassend eine erste flüssige mobile Phase und eine flüssige stationäre Phase, enthaltend mindestens eine Austauscher-Substanz, die ablösbar an das Ziel-Oligonukleotid bindet;
das Bewirken, dass die erste flüssige mobile Phase das Ziel-Oligonukleotid in einem Fluss relativ zu und in Kontakt mit der flüssigen stationären Phase in der Säule eines Flüssigkeits - Flüssigkeitschromatographieapparats trägt, so dass das Ziel-Oligonukleotid an die Austauscher-Substanz in der flüssigen stationären Phase gebunden wird;
dann das Verdrängen des Ziel-Oligonukleotids aus der flüssigen stationären Phase in eine zweite flüssige mobile Phase, die relativ zu und in Kontakt mit der stationären Phase durch die Säule fließt, mittels einer Verdränger-Substanz, die fähig ist, das Ziel-Oligonukleotid aus der flüssigen stationären Phase in die zweite mobile Phase zu verdrängen,
**dadurch gekennzeichnet, dass** die Austauscher-Substanz ein Salz eines organischen sekundären, tertiären oder quartären Amins mit einem Gegenanion ist, mit der allgemeinen Formel:
R¹R²R³R⁴N⁺X⁻
wobei durch die Sequenz hindurch sekundäre, tertiäre und quartäre beziehungsweise zwei, drei oder vier der Gruppen R¹, R², R³ und R⁴ unabhängig C₁₋₂₀-Alkyl oder substituiertes Alkyl, wie beispielsweise Fluor- oder Trifluormethyl-substituiertes Alkyl, oder Benzyl sind, und die Übrigen Wasserstoff sind, und X- ein Halidanion ist.

2. Verfahren gemäß Anspruch 1, **gekennzeichnet durch** die Schritte des:
(1) Bereitstellens einer ersten flüssigen Phase, enthaltend das Ziel-Oligonukleotid und eine oder mehrere Verunreinigung(en) in Lösung, und eine zweite flüssige Phase, die die Austauscher-Substanz, die ablösbar an das Ziel-Oligonukleotid bindet, enthält; die erste und zweite flüssige Phase zwei verschiedene Phasen bildend, wenn sie miteinander in Kontakt sind;
(2) Einbringens der zweiten flüssigen Phase in einen Zentrifugal-Verteilungschromatographie-Apparat als eine stationäre flüssige Phase darin;
(3) Einbringens der ersten flüssigen Phase, die das Ziel-Oligonukleotid und eine oder mehrere Verunreinigung(en) in Lösung enthält, in den Zentrifugal-Verteilungschromatographie-Apparat als eine erste mobile Phase und des Bewirkens, dass diese erste flüssige Phase **durch** den Zentrifugal-Verteilungschromatographie-Apparat in Kontakt mit der zweiten flüssigen stationären Phase fließt, so dass das Ziel-Oligonukleotid ablösbar an die Austauscher-Substanz in der zweiten flüssigen stationären Phase gebunden wird;
(4) Einbringens einer dritten flüssigen Phase, die eine verschiedene Phase bildet, wenn sie in Kontakt mit der zweiten flüssigen Phase ist, und die in Lösung mindestens eine Verdränger-Substanz enthält, die fähig ist, das Ziel-Oligonukleotid aus der zweiten flüssigen Phase in den Zentrifugal-Verteilungschromatographie-Apparat zu verdrängen, als eine zweite mobile Phase und des Bewirkens, dass diese zweite mobile Phase **durch** den Zentrifugal-Verteilungschromatographie-Apparat in Kontakt mit der zweiten flüssigen Phase fließt, so dass das Ziel-Oligonukleotid aus der stationären Phase verdrängt wird und in der zweiten mobilen Phase in Lösung eintritt;
(5) Isolierens des verdrängten Ziel-Oligonukleotids aus der zweiten mobilen Phase.

3. Verfahren gemäß Anspruch 1 oder 2, **dadurch gekennzeichnet, dass** das Ziel-Oligonukleotid 10 - 30 Basen umfasst.

4. Verfahren gemäß Anspruch 3, **dadurch gekennzeichnet, dass** das Ziel-Oligonukleotid das 20-Basen-Oligonukleotid ist, das die Sequenz 5'-UCAAGGAAGAUGGCAUUUCA-3' hat.

5. Verfahren gemäß irgendeinem der vorstehenden Ansprüche, **dadurch gekennzeichnet, dass** die stationäre Phase eine Mischung aus einer oder mehreren organischen Flüssigkeit(en), die im Wesentlichen mit Wasser unmischbar ist/sind, und einer oder mehreren organischen Flüssigkeit(en), die mit Wasser mischbar ist/sind, umfasst.

6. Verfahren gemäß Anspruch 5, **dadurch gekennzeichnet, dass** die erste und zweite mobile Phase eine Mischung aus einer oder mehreren organischen Flüssigkeit(en), die mit Wasser mischbar ist/sind, und Wasser umfassen.

7. Verfahren gemäß Anspruch 5 oder 6, **dadurch gekennzeichnet, dass** die stationären und mobilen Phasen die zwei jeweiligen Gleichgewichtsphasen umfassen von: (i) einem flüssigen System, das C₁₋₆-Alkyl-C₁₋₆-Alkanoat-Ester, C₁₋₈-Alkanol und Wasser enthält; oder (ii) einem flüssigen System, das C₁₋₈-Alkanol und Wasser enthält; oder (iii) einem flüssigen System, das C₁₋₈-Alkanol, Di-(C₁₋₈-Alkyl)-Keton und Wasser enthält; oder (iv) einem flüssigen System, das Di-(C₁₋₈-Alkyl)-Keton und Wasser enthält; oder (v) einem flüssigen System, das C₁₋₆-Alkyl-C₁₋₆-Alkyl-Ether oder einen C₄₋₁₀-zyklischen-Ether, C₁₋₈-Alkanol und Wasser enthält.

8. Verfahren gemäß irgendeinem der vorstehenden Ansprüche, **dadurch gekennzeichnet, dass** das Halidanion der Austauscher-Substanz Chlorid ist.

9. Verfahren gemäß Anspruch 8, **dadurch gekennzeichnet, dass** die Austauscher-Substanz ausgewählt ist aus einer Mischung aus Tri-(n-Octyl)-Methyl-Ammoniumchlorid und Tri-(n-Decyl)-Methyl-Ammoniumchlorid; und Benzalkoniumchlorid.

10. Verfahren gemäß irgendeinem der Ansprüche 1 bis 7, **dadurch gekennzeichnet, dass** die Austauscher-Substanz ausgewählt ist aus Cetyltrimethylammoniumbromid, Methyltrioctylammoniumchlorid, einer Mischung aus Alkylbenzyldimethylammoniumchloriden mit vielfältigen Alkyl-Kettenlängen, Benzyltrimethylammoniumchlorid, Tetrabutylammoniumchlorid, und einem öllöslichem sekundärem Amin mit hohem Molekulargewicht, das als eine Flüssigkeit in der freien-Base-Form in protonierter Form bereitgestellt wird.

11. Verfahren gemäß irgendeinem der vorstehenden Ansprüche, **dadurch gekennzeichnet, dass** die zweite flüssige mobile Phase die gleiche Zusammensetzung der Flüssigkeiten wie die erste mobile Phase hat.

12. Verfahren gemäß Anspruch 11, **dadurch gekennzeichnet, dass** die stationäre Phase eine Mischung aus überwiegend C₁₋₈-Alkanol und C₁₋₆-Alkyl-C₁₋₆-Alkanoat-Ester umfasst, und die zweite mobile Phase eine Mischung aus Wasser und C₁₋₈-Alkanol umfasst.

13. Verfahren gemäß Anspruch 11, **dadurch gekennzeichnet, dass** die stationäre Phase eine Mischung aus überwiegend C₁₋₆-zyklischem-Ether und C₁₋₈-Alkanol umfasst, und die zweite mobile Phase eine Mischung aus überwiegend Wasser und C₁₋₈-Alkanol umfasst.

14. Verfahren gemäß irgendeinem der vorstehenden Ansprüche, **dadurch gekennzeichnet, dass** die Verdränger-Substanz ausgewählt ist aus Alkalimetall-Haliden, Sulphaten und Oxalaten, Saccharin-Natriumsalz, Sonnenuntergangs-Gelb ("Sunset Yellow) und Amarant Amaranth").

15. Verfahren gemäß irgendeinem der vorstehenden Ansprüche, **dadurch gekennzeichnet, dass** die Konzentration der Verdränger-Substanz in der zweiten mobilen Phase 5-30 mM ist.

## Revendications

1. Procédé de séparation d'un oligonucléotide cible à partir d'un mélange contenant l'oligonucléotide cible et une ou plusieurs impuretés, comprenant :
la fourniture d'un système de chromatographie liquide/liquide biphasique à phase mobile/phase stationnaire comprenant une première phase liquide mobile, et une phase liquide stationnaire contenant au moins une substance échangeuse qui se lie de manière détachable à l'oligonucléotide cible ;
le fait d'amener la première phase liquide mobile à transporter l'oligonucléotide cible dans un écoulement par rapport à et en contact avec la phase liquide stationnaire dans une colonne d'un appareil de chromatographie liquide/liquide de façon que l'oligo-nucléotide cible se lie à la substance échangeuse dans la phase liquide stationnaire ;
puis le déplacement de l'oligonucléotide cible à partir de la phase liquide stationnaire dans une seconde phase liquide mobile s'écoulant par rapport à et en contact avec la phase stationnaire à travers la colonne au moyen d'une substance de déplacement capable de déplacer l'oligonucléotide cible depuis la phase liquide stationnaire vers la seconde phase mobile,
**caractérisé en ce que** la substance échangeuse est un sel d'une amine organique secondaire, tertiaire ou quaternaire avec un contre-anion, de formule général
R¹R²R³R⁴N⁺X⁻
dans laquelle, selon l'ordre secondaire, tertiaire et quaternaire, respectivement deux, trois ou quatre des groupes R¹, R², R³ et R⁴ sont indépendamment un alkyle en C₁ à C₂₀ ou un alkyle substitué, tel qu'un fluoroalkyle ou alkyle substitué avec un trifluorométhyle, ou un benzyle, et les autres sont des hydrogènes, et X⁻ est un anion halogénure.

2. Procédé selon la revendication 1, **caractérisé par** les étapes suivantes :
(1) la fourniture d'une première phase liquide contenant l'oligonucléotide cible et une ou plusieurs impuretés en solution, et d'une deuxième phase liquide contenant la substance échangeuse qui est liée de manière détachable à l'oligonucléotide cible, les première et deuxième phases liquides formant deux phases distinctes quand elles sont en contact l'une avec l'autre ;
(2) l'introduction de la deuxième phase liquide dans un appareil de chromatographie de partage par centrifugation en tant que phase liquide stationnaire :
(3) l'introduction de la première phase liquide contenant l'oligonucléotide cible et une ou plusieurs impuretés en solution dans l'appareil de chromatographie de partage par centrifugation en tant que première phase mobile et le fait d'amener cette première phase liquide à s'écouler à travers l'appareil de chromatographie de partage par centrifugation en contact avec la deuxième phase liquide stationnaire de façon à ce que l'oligonucléotide cible se lie de manière détachable à la substance échangeuse dans la deuxième phase liquide stationnaire ;
(4) l'introduction d'une troisième phase liquide qui forme une phase distincte quand elle est en contact avec la deuxième phase liquide et qui contient en solution au moins une substance de déplacement capable de déplacer l'oligonucléotide cible depuis la deuxième phase liquide dans l'appareil de chromatographie de partage par centrifugation, en tant que seconde phase mobile et le fait d'amener cette seconde phase mobile à s'écouler à travers l'appareil de chromatographie de partage par centrifugation en contact avec la deuxième phase liquide de façon que l'oligonucléotide cible soit déplacé depuis la phase stationnaire et entre en solution dans la seconde phase mobile ;
(5) l'isolement de l'oligonucléotide cible déplacé à partir de la seconde phase mobile.

3. Procédé selon la revendication 1 ou 2, **caractérisé en ce que** l'oligonucléotide cible comprend 10 à 30 bases.

4. Procédé selon la revendication 3, **caractérisé en ce que** l'oligonucléotide cible est l'oligonucléotide de 20 bases qui possède la séquence 5'-UCAAGGAAGAUGGCAUUUCA-3'.

5. Procédé selon l'une quelconque des revendications précédentes, **caractérisé en ce que** la phase stationnaire comprend un mélange contenant un ou plusieurs liquides organiques qui sont sensiblement non miscibles à l'eau et un ou plusieurs liquides organiques qui sont miscibles à l'eau.

6. Procédé selon la revendication 5, **caractérisé en ce que** les première et seconde phases mobiles comprennent un mélange contenant un ou plusieurs liquides organiques qui sont miscibles à l'eau, et de l'eau.

7. Procédé selon la revendication 5 ou 6, **caractérisé en ce que** les phases stationnaires et mobiles comprennent les deux phases respectives à l'équilibre : (i) d'un système liquide contenant un ester d'alcanoate en C₁ à C₆ d'alkyle en C₁ à C₆, un alcanol en C₁ à C₈ et de l'eau ; ou (ii) d'un système liquide contenant un alcanol en C₁ à C₈ et de l'eau ; ou (iii) d'un système liquide contenant un alcanol en C₁ à C₈, une di-(alkyle en Ci à C₈)cétone et de l'eau ; ou (iv) d'un système liquide contenant une di-(alkyle en C₁ à C₈)cétone et de l'eau ; ou (v) d'un système liquide contenant un (alkyle en C₁ à C₆)(alkyle en C₁ à C₆)éther ou un éther cyclique en C₄ à C₁₀, un alcanol en C₁ à C₈ et de l'eau.

8. Procédé selon l'une quelconque des revendications précédentes, **caractérisé en ce que** l'anion halogénure de la substance échangeuse est le chlorure.

9. Procédé selon la revendication 8, **caractérisé en ce que** la substance échangeuse est choisie parmi un mélange de chlorure de tri-(n-octyl)méthylammonium et de chlorure de tri-(n-décyl)méthylammonium ; et le chlorure de benzalkonium.

10. Procédé selon l'une quelconque des revendications 1 à 7, **caractérisé en ce que** la substance échangeuse est choisie parmi le bromure de cétyltriméthylammonium, le chlorure de méthyltrioctyl-ammonium, un mélange de chlorures d'alkylbenzyldiméthyl ammonium présentant diverses longueurs de chaîne alkyle, le chlorure de benzyltriméthylammonium, le chlorure de tétrabutylammonium, et une amine secondaire oléosoluble de poids moléculaire élevé fournie sous la forme d'un liquide de la base libre sous forme protonée.

11. Procédé selon l'une quelconque des revendications précédentes, **caractérisé en ce que** la seconde phase liquide mobile a la même composition de liquides que la première phase mobile.

12. Procédé selon la revendication 11, **caractérisé en ce que** la phase stationnaire comprend un mélange contenant principalement un alcanol en C₁ à C₈ et un ester d'alcanoate en C₁ à C₆ d'alkyle en C₁ à C₆, et la seconde phase mobile comprend un mélange contenant de l'eau et un alcanol en C₁ à C₈.

13. Procédé selon la revendication 11, **caractérisé en ce que** la phase stationnaire comprend un mélange contenant principalement un éther cyclique en C₁ à C₆ et un alcanol en C₁ à C₈, et la seconde phase mobile comprend un mélange contenant principalement de l'eau et un alcanol en C₁ à C₈.

14. Procédé selon l'une quelconque des revendications précédentes, **caractérisé en ce que** la substance de déplacement est choisie parmi les halogénures, sulfates et oxalates de métaux alcalins, un sel de saccharine sodique, le jaune-orange S et le l'amaranthe.

15. Procédé selon l'une quelconque des revendications précédentes, **caractérisé en ce que** la concentration de la substance de déplacement dans la seconde phase mobile est de 5 à 30 mM.
